Europäisches Patentamt

European Patent Office

Office européen des brevets

(11) Veröffentlichungsnummer : **0 166 860**
**B1**

(12)

# EUROPÄISCHE PATENTSCHRIFT

(45) Veröffentlichungstag der Patentschrift :
17.05.89

(21) Anmeldenummer : 85102375.4

(22) Anmeldetag : 02.03.85

(51) Int. Cl.⁴ : **A 61 B   1/00**, H 01 R   4/60

(54) **Lichtleitereinkopplung für ein medizinisches Lasergerät.**

(30) Priorität : 01.06.84 DE 8416748 U

(43) Veröffentlichungstag der Anmeldung :
08.01.86 Patentblatt 86/02

(45) Bekanntmachung des Hinweises auf die Patenterteilung : 17.05.89 Patentblatt 89/20

(84) Benannte Vertragsstaaten :
AT BE CH DE FR GB IT LI LU NL SE

(56) Entgegenhaltungen :
EP—A— 0 088 360
DE—A— 2 948 564

(73) Patentinhaber : **Messerschmitt-Bölkow-Blohm Gesellschaft mit beschränkter Haftung**
**Robert-Koch-Strasse**
**D-8012 Ottobrunn (DE)**

(72) Erfinder : **Hengst, Thomas**
**Wieselweg 23**
**D-8013 Haar (DE)**
Erfinder : **Hahn, Andreas**
**Ringbergstrasse 19**
**D-8029 Sauerlach (DE)**

EP 0 166 860 B1

## Beschreibung

Die Neuerung betrifft eine Lichtleitereinkopplung für ein medizinisches Lasergerät mit einer hülsenförmigen Fassung zum Anschluß an einen Laser.

Für die Anwendung von Lasern im medizinischen oder technischen Bereich werden Kupplungen zwischen dem Laser selbst und dem Applikationsgerät benötigt, die neben der Weiterleitung des Laserlichtstrahles auch eine elektrische Verbindung und die Weiterleitung eines Gasstromes vollziehen. Bisher war es dabei üblich, jeweils separate Kopplungselemente für Laserstrahl, Strom und Gas zu verwenden, was zu aufwendigen Konstruktionen führte, wobei eine fehlerfreie Bedienung der Kopplungselemente bei der Handhabung nicht immer ausgeschossen war.

Der Neuerung liegt deshalb die Aufgabe zugrunde, eine Lichtleitereinkopplung für ein medizinisches Lasergerät so auszubilden, daß neben der optischen Kupplung gleichzeitig auch eine Gaszuführung und elektrische Kontakte angeschlossen werden, über welche sowohl der Verschluß des Lasergerätes gesteuert als auch eine anwendungsbezogene Codierung in Abhängigkeit vom verwendeten Applikator bzw. Lichtleitertyp vollzogen wird.

Diese Aufgabe wird neuerungsgemäß durch die im kennzeichnenden Teil des Anspruches wiedergegebenen Merkmale gelöst.

Ein Ausführungsbeispiel ist in der Zeichnung dargestellt und wird im folgenden näher beschrieben. Es zeigen :

Fig. 1 einen schematisch vereinfachten Längsschnitt durch eine Lichtleitereinkopplung ;

Fig. 2 eine Frontansicht der Lichtleitereinkopplung.

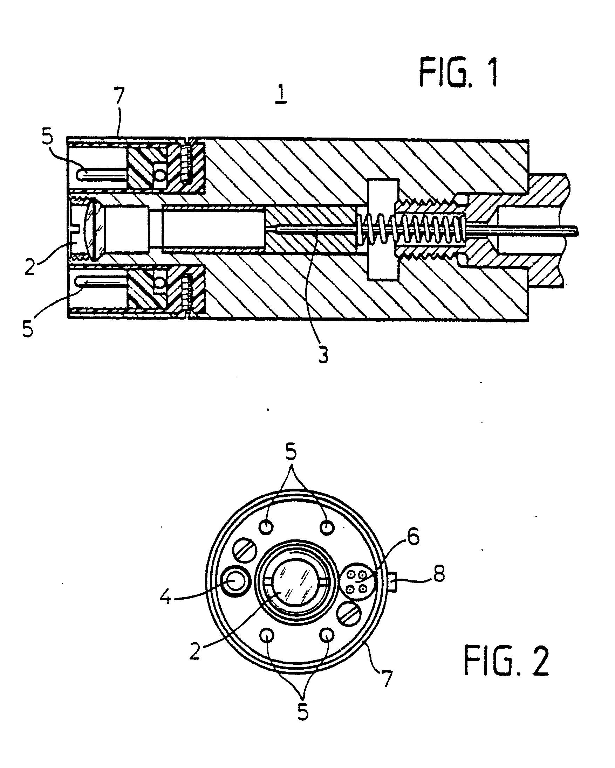

Die Fig. 1 zeigt ein Beispiel einer neuerungsgemäßen Lichtleitereinkopplung 1. Axial zentrisch ist die optische Kupplung 2 des Lichtleiters 3 dargestellt, der mittels einer — hier nicht dargestellten — Einrichtung in die Lichtleitereinkopplung eingeführt wird. Radial um die optische Kupplung sind die weiteren benötigten Anschlüsse angeordnet. Zunächst ist eine Kupplung 4 für eine Gasleitung vorgesehen, über welche das zur Spülung des Lichtleiters benötigte Gas herbeigeführt wird.

Weiterhin ist eine Gruppe von elektrischen Kontakten 5 vorgesehen, die auch mit größeren Stromstärken zum Schalten von Relais oder zum Ansteuern von Verbrauchern belastet werden können. Im vorliegenden Beispiel werden diese Kontakte dazu benutzt, um als Kurzschlußstecker ein automatisches Schließen des Laser-Verschlusses zu gewährleisten, wenn der über die Lichtleitereinkopplung 1 betriebene Applikator abgesteckt wird.

Zusätzlich zur ersten Gruppe von elektrischen Kontakten 5 ist eine weitere Gruppe von elektrischen Kontakten 6 eingebaut, die nur für eine geringe Strombelastbarkeit, z. B. für die Übertragung von Daten, ausgelegt sind.

In dem in Fig. 2 gezeigten Beispiel sind die Kontakte 6 in der Form eines Quadrates angeordnet, um so ein verwechslungsfreies Anstecken der Lichtleitereinkopplung 1 an einen Applikator zu ermöglichen. Die Kontakte 6 dienen hier als Programmier-Stecker zur Identifikation des jeweiligen Applikatortyps und zur Codierung des angeschlossenen Lichtleitertyps. Somit wird bei dem Anschluß eines nur wenig belastbaren Lichtleiters automatisch die Lichtleistung des Lasers gedrosselt und damit eine Überlastung vermieden. Ebenso können über die Kontakte 6 anwendungsspezifische Daten, wie z. B. der benötigte Gasfluß oder sonstige technisch/physikalische Größen des angeschlossenen Applikators an den Laser weitergegeben werden.

Den äußeren Abschluß der Lichtleitereinkopplung 1 bildet ein hülsenförmiger Mantel 7 aus Metall, der zu den übrigen Teilen der Lichtleitereinkopplung elektrisch isoliert ist. Dieser Mantel 7 kann im Bedarfsfall Verriegelungselemente 8 aufweisen, mittels denen eine mechanische Ankopplung und Verriegelung der Lichtleitereinkopplung an einen Applikator nach der Art eines Bajonettverschlusses vollzogen wird.

Der besondere Vorteil der Neuerung ist darin zu sehen, daß eine einstückige Lichtleitereinkopplung für medizinische Lasergeräte geschaffen wurde, die gleichzeitig ein verwechslungsfreies und gefahrloses Ankoppeln eines Applikators ermöglicht, eine Gaszufuhr anschließt und über elektrische Kontakte sowohl den Verschluß des Lasergerätes als auch die Lichtleistung des Lasers und weitere anwendungsspezifische Größen steuert.

## Patentanspruch

Lichtleitereinkopplung (1) für ein medizinisches Lasergerät mit einer hülsenförmigen Fassung zum Anschluß an einen Laser, dadurch gekennzeichnet, daß an einem zylindrischen Kupplungsstück um die zentrisch eingebaute optische Kupplung (2) für den Lichtleiter (3) radial weitere Verbindungselemente angeordnet sind :

    a) eine Kupplung (4) für eine Gaszuleitung,

    b) eine Vielzahl von symmetrisch zur optischen Kupplung (2) positionierten elektrischen Kontakten (5) mit höherer Strombelastbarkeit,

    c) eine Vielzahl von elektrischen Kontakten (6) zur Datenübertragung, wobei die Anordnung der einzelnen Kontakte zueinander willkürlich wählbar ist,

    d) einen hülsenförmigen Mantel (7), der gegebenenfalls mit Verriegelungselementen (8) ausgerüstet ist.

## Claim

A photoconductor coupling device (1) for a medical laser instrument with a sleeve-shaped holder for the connection to a laser, characterised in that on a cylindrical coupling piece about the centrally installed optical coupling (2) for the photoconductor (3) further connection elements are radially arranged :

    a) a coupling (4) for a gas supply-pipe,

    b) a plurality of electrical contacts (5) having higher current loadability, positioned symmetrically with regard to the optical coupling (2),

    c) a plurality of electrical contacts (6) for the data transmission, in which respect the arrangement of the individual contacts with regard to one another is arbitrarily selectable,

    d) a sleeve-shaped jacket (7), which is equipped with locking elements (8).


**Revendication**

Dispositif de couplage de fibre optique (1) pour un appareil laser médical, comprenant une monture en forme de douille pour le raccordement à un laser, caractérisé en ce que sur une pièce de couplage cylindrique, des éléments de liaison supplémentaires sont disposés radialement autour du coupleur optique (2) central pour la fibre optique (3), à savoir

    a) un coupleur (4) pour une amenée de gaz,

    b) une multitude de contacts électriques (5) placés de façon symétrique par rapport au coupleur optique (2) et admettant une intensité de courant maximale élevée,

    c) une multitude de contacts électriques (6) pour la transmission de données, l'agencement des contacts individuels les uns par rapport aux autres pouvant être choisi arbitrairement,

    d) une enveloppe (7) en forme de douille qui est équipée le cas échéant d'éléments de verrouillage (8).

FIG. 1

FIG. 2